# EUROPEAN PATENT SPECIFICATION

(11) **EP 3 532 849 B1**
(45) Date of publication and mention of the grant of the patent: **01.06.2022**
(21) Application number: 17801361.1
(22) Date of filing: 31.10.2017
(51) Int. Cl.: G01N 33/569, G01N 33/543, G01N 33/68

(54) **DEVICE AND METHOD FOR DETECTING A BACTERIAL INFECTION**
VORRICHTUNG UND VERFAHREN ZUM NACHWEIS EINER BAKTERIELLEN INFEKTION
DISPOSITIF ET PROCÉDÉ POUR DÉTECTER UNE INFECTION BACTÉRIENNE

(30) Priority: 31.10.2016 SE 1651433
(43) Date of publication of application: 04.09.2019
(73) Proprietor: PEAS Institut AB, 587 23 Linköping (SE)
(72) Inventor: NAYERI, Fariba, 587 37 Linköping (SE)
(74) Representative: Brann AB
(86) International application number: PCT/EP2017/077916
(87) International publication number: WO 2018/078184

(56) References cited:
- WO-A1-2010/151222
- WO-A1-2012/156465
- WO-A1-2013/126013
- SRAVYA SOWDAMINI NAKKA ET AL: "A methachromatic-based experimental model for identification of bowel as the focus of an acute inflammation", OPEN JOURNAL OF GASTROENTEROLOGY, vol. 03, no. 01, 1 January 2013 (2013-01-01), pages 42-48, XP055199165, ISSN: 2163-9450, DOI: 10.4236/ojgas.2013.31007
- ASHRAF E. SOROUR ET AL: "Evaluation of hepatocyte growth factor as a local acute phase response marker in the bowel: The clinical impact of a rapid diagnostic test for immediate identification of acute bowel inflammation", CYTOKINE, vol. 71, no. 1, 1 January 2015 (2015-01-01), pages 8-15, XP055440158, US ISSN: 1043-4666, DOI: 10.1016/j.cyto.2014.07.255
- S. P. YAZDANKHAH ET AL: "Rapid Method for Detection of Gram-Positive and -Negative Bacteria in Milk from Cows with Moderate or Severe Clinical Mastitis", JOURNAL OF CLINICAL MICROBIOLOGY, vol. 39, no. 9, 1 September 2001 (2001-09-01), pages 3228-3233, XP055440161, US ISSN: 0095-1137, DOI: 10.1128/JCM.39.9.3228-3233.2001
- KELER T ET AL: "Metachromatic assay for the quantitative determination of bacterial endotoxins", ANALYTICAL BIOCHEMISTRY, ELSEVIER, AMSTERDAM, NL, vol. 156, no. 1, 1 July 1986 (1986-07-01), pages 189-193, XP024817247, ISSN: 0003-2697, DOI: 10.1016/0003-2697(86)90172-7 [retrieved on 1986-07-01]
- Thomas Hahn ET AL: "Dye affinity chromatography for fast and simple purification of fucoidan from marine brown algae", ENGINEERING IN LIFE SCIENCES, vol. 16, no. 1, 13 October 2015 (2015-10-13), pages 78-87, XP055735431, DE ISSN: 1618-0240, DOI: 10.1002/elsc.201500044
- Yvette M. Coulson-Thomas ET AL: "The Identification of Proteoglycans and Glycosaminoglycans in Archaeological Human Bones and Teeth", PLOS ONE, vol. 10, no. 6, 24 June 2015 (2015-06-24), page e0131105, XP055453694, DOI: 10.1371/journal.pone.0131105

## Description

### TECHNICAL FIELD OF THE INVENTION

The present invention relates to the field of diagnosis and more particularly to determining the presence or absence of a bacterial infection in an individual by detecting proteins produced by said individual in response to a bacterial infection. It also relates to determining the severity of and/or monitoring a bacterial infection in said individual.

### BACKGROUND OF THE INVENTION

An infection occurs when invading pathogenic microorganisms gain a foothold, succeed in growing, and thereby disturb the normal physiological properties of the tissue [Peterson JW, 1996]. To minimize injuries to the patient, the microbe responsible for the infection must be identified, its susceptibility to antibiotics determined, and effective therapy initiated immediately [Tunkel AR,p.1267, 2004]. The discovery and implementation of antibiotics is tremendously valuable in targeting specific pathogens, effectively eliminating them, and saving lives and resources [Infectious Diseases Society of America (IDSA), S397, 2011]. However, sufficient guidelines and proper diagnostic instruments are not always available to communities to support proper antibiotics administration. Insufficient compliance regarding intake and unregulated administration has, in part, resulted in development of resistant bacterial strains [Pechere JC, p.245,2007]. Among the factors that promote the global dissemination of antimicrobial resistance is the increasing international travel and extensive intercontinental commerce. The current circumstances in developing regions of the world also contribute to the spread of antimicrobial resistance-something that is undoubtedly related to population density, household purchasing power, drug availability, over-the-counter availability of antibiotics, hygiene and sanitation standards, and organized disease control and prevention [Bonelli RR, p.24,2014]. Antibiotic resistance rates in Gram-negative bacteria are rapidly increasing, especially with regard to resistance to third and fourth generation cephalosporin and mainly due to the production of extended-spectrum β-lactamases (ESBLs) (Falagas, p.345-354, 2009). Infections caused by such enzyme-producing organisms are associated with a higher morbidity and mortality and greater fiscal burden (Richi H, 625170, 2012.). The prevalence of ESBLs varies worldwide, with reports from North America, South America, Europe, Africa, and Asia. Data from the global surveillance database shows the rate of ESBL production was highest among the *Klebsiella pneumoniae* isolates collected in Latin America, followed by Asia/Pacific Rim, Europe, and North America (44.0%, 22.4%, 13.3%, and 7.5%, resp.) (Reinert, p. 1018-1029, 2007). The incidence of ESBL infections per 10,000 discharges increased for both healthcare-associated infections, 1.9 per year (95% CI 1-2.8), and for community-acquired infections, 0.85 per year (95% CI 0.3-1.4) as reported in a recent study from USA (Kassakian, p. 9, 2014). *Pseudomonas aeruginosa* is an important pathogen frequently implicated in healthcare-associated infections, particularly in critically ill or immunocompromised patients (Hirsch, p. 441-451, 2010). Broad spectrum antimicrobial resistance in isolates significantly limits effective therapeutic options (Oliver, p.41-59, 2015). ESBL-producing Salmonella isolates resistant to several antibiotic agents, especially 3rd generation cephalosporin, are increasingly reported (Uma, p.201-204, 2010). In recent years, studies have suggested that international travel and/or gastroenteritis during travel is a risk factor for infection with multiple resistant ESBL producing enterobacteriaceae (Ostholm-Balkhed, p.2144-53, 2013).

Urinary tract infection (UTI) remains to be the most common infection diagnosed in outpatients as well as in hospitalized patients. The studies have revealed a global high frequency of antimicrobial resistance and ESBL production in Enterobacteriaceae isolated from urine samples in patients (Cohen-Nahum p.41-6, 2010) .The rapid evolution and spread of antimicrobial-resistant bacteria in parallel with insufficient development of new active drugs seriously affect future anti-infective therapy of bacterial infections due to Gram-negative rods in the most common diseases; infectious gastroenteritis and urinary tract infection. Experts in the field estimate that by the next decade the world will have witnessed the wide dissemination of untreatable infections, both within and beyond hospitals (Laxminarayan R, p. 1057-98, 2013). Upper respiratory tract infections are a major reason for the prescription of antibiotics, even though many of these infections are due to viruses, where antibiotics are neither effective nor necessary.

Efforts in order to decrease the antibiotic consumption might have an impact to control the world-wide problem of emerging multiple resistant microorganisms (Nielsen, p. 28-42, 2015). Therefore an effective approach to controlling the problem is directed antibiotic therapy [Gaieski DF, p. 1045,2010], which requires developing methods to diagnose an infection and identify the microorganism prior to initiating therapy. Laboratory culturing methods and highly technological PCR-based assays that are employed to identify pathogenic microorganisms plus their susceptibility to specific antimicrobial agents are of immense value [Kerremans JJ, p.428, 2008]. But, to fully determine the need for antibiotic therapy, distinguishing harmless colonization of pathogens from active infection is crucial. Herein lays a certain level of complexity that needs unraveling because the host environment houses different constellations of pathogenic microorganisms that, together in symbiosis, can produce synergistic survival mechanisms called biofilms [SteartPS, p.135, 2001]. In polymicrobial environments, slow-growing bacteria constitute infections that are difficult to detect due to rapidly-growing microorganisms whose rate of growth obscures the true result in culture. Using even the latest PCR methods, the multiple outcomes confuse practitioners [Wolcott R, p 107, 2013; Melendez JH, p. 1762, 2010]. Therefore, tools to assess the inflammatory reaction and response of tissue to the presence of corrosive pathogens are urgently needed. Direct examination of the body fluids such as stool for faecal leukocytes has been used for decades as an indicator of intestinal inflammation [Harris JC, 1972]. The methodology is simple enough that someone with microscopy skills and a very basic laboratory can perform the test reliably. The finding of >10 leukocytes per HPF is considered positive for infection.

Markers with short half-lives provide a recent picture of organ status including recovery. Acute phase reactants, C-reactive protein and procalcitonin have been studied widely and are acknowledged by physicians as tools for indicating and monitoring acute injury, including those sustained by infection [Pepys MB, p. 1805, 2003; Xiao Z , p. 1093, 2015; Lam SW, 2015]. However, these markers are analyzed systemically and cannot locate the focus of the infection, especially during hazardous sub-acute and silent infections.

The extracellular matrix (ECM) is composed of molecules produced by the cells in organ tissues that provide structural and biochemical support to the surrounding cells [Theocharis AD, 2015]. By studying the physiological aspects of the ECM in healthy persons, alterations found in its counterparts and products might reflect disease. Heparan sulfate proteoglycan (HSPG) is an important component of the ECM that, inter alia, is involved in the activation and elimination of cytokines and growth factors produced by various cells during injury [Nadanaka S, p.7, 2008]. One such well-studied growth factor is hepatocyte growth factor (HGF), which is known as a multifunctional regenerative factor that is delivered endocrinally (systemic) as well as paracrinally (local) to injured epithelial cells [Molnarfi N , p.293, 2015; Nakamura T, p.188, 2011]. Production of biologically-active HGF increased during acute bacterial infections [Nayeri F, p.13, 2005; Nayeri F, p.2092, 2000]. The binding profile to HSPG in body fluids could reflect the pathophysiological status of injured organs, identify an acute inflammation due to microbial invasion, and differentiate acute from chronic inflammation [Lönn J, p.33, 2013].

Proteins can be detected based on their specific interaction with a corresponding antibody. However, this measurement system relies on specialized resources, limiting its usefulness in non-equipped centers or as a self-test. Methachromasy is a characteristic color change exhibited by certain aniline dyes upon binding to chromotropic substances [Bergeron JA, 1958]. This phenomenon has been widely used in histology. Methylene blue (O-Toluidine) is an excellent metachromatic dye that changes from blue to pink upon binding to high-molecular-weight polysaccharides, such as sulfated glycan [Pham NA, 2007]. The pink dye will then quickly turn back to blue following addition of a proportional amount of a protein with high affinity to sulfated glycan (inverted methachromacy).

WO2010/151222 and S. Sowdamini Nakka et al. (Open Journal of Gastroenterology, 2013, 3, 42-48) concern a system for determining the presence, absence or amount of hepatocyte growth factor (HGF) in a sample comprising adding said sample to a gel containing a HGF-binding component, adding toluidine blue to said gel and thereafter correlating the colour of the gel with the amount of HGF in said sample. The amount of HGF is an indicator of the presence of an acute inflammation.

Ashraf E. Sorour et al. (Cytokine, vol.71, nov.1, 1 January 2015, p.8 to 15) disclose a rapid diagnostic test for distinguishing contagious gastroenteritis from exacerbation of chronic inflammatory bowel disease (IBD) or boewl engagement in the course of systemic inflammatory response syndrome SIRS. The test strip detects the presence of HGF as a local acute phase response marker in the bowel. Fecal pH is used as an indicator of illness severity.

WO2013126013 describes a test strip for detecting the presence of HGF in a sample, said test strip comprising one or two surfaces, wherein one surface contains a HGF-binding component and an indicator solution comprising bromothymol blue and a quaternary ammonium compound and the second surface comprises a pH indicator dye.

WO2012/156456 describes a method or a system for diagnosing a Th1 or Th2 mediated disease by contacting a sample of a body fluid of said patient with at least one redox indicator dye at a specific pH value. A colour change will occur resulting from the direct reaction of the redox indicator dye with a metabolite in the body fluid sample.

S.P. Yazdankhah et al (Journal of clinical microbiology, vol. 39, no.9, 1 September 2001, p.3228-3233) describes a method and a device for detecting mastitis, said method making it possible to differentiate gram positive bacteria from gram negative bacteria present in a milk sample using a polysulfone filter soaked with toluidine blue.

We have previously in several studies shown that amounts and biological activity of Hepatocyte Growth Factor (HGF) could be used for diagnosis of acute inflammation in body fluids. However, there still remains a need to further develop and sensitize the diagnostic methods for bacterial infections available today. There is also a need for faster, more reliable measures for monitoring therapy, such as antibiotic therapy, of bacterial infections.

### SUMMARY OF THE INVENTION

The above problems and drawbacks have now been overcome, or at least mitigated, by a device and an *in vitro* method provided herein. Hence herein, we present a test (or a kit) comprising a device according to the present disclosure to diagnose a bacterial infection by the determination of acute phase proteins in an individual e.g. making it possible to monitor an ongoing antibiotic therapy administered in less than 36 hours. The test may be performed via analysis of biological samples comprising body fluids without any preparations, in less than 5 minutes and less than 0.4 GBP cost per test.

The testing using said device might be performed by the patient, relatives or the health workers throughout the world and the results can rule out bacterial infection or therapy failure with a 98% negative predictive value. The test is designed to be environmentally harmless, low weight, stable to temperature or mechanical strains and has over 2 years shelf-life. The range between positive (sky blue) and negative result (horizon red) is detectable by naked eye (normal vision) in daylight and has the capacity to give quantitative values using spectrophotometry or RGB Reader (Red Green Blue Reader). Using the test might result in huge decrease in social and individual costs and an objective antibiotic treatment strategy. Daily monitoring can detect a therapy failure due to antibiotic resistance within few days and therefore the high technical resources to identify antibiotic resistant genes can be used in the few but reasonable cases.

Accordingly, there is provided herein a device for quantitatively determining the presence or absence of one or more acute phase protein(s), such as Hepatocyte Growth Factor (HGF), in a biological sample from an individual, wherein a presence of one or more acute phase protein(s) in said biological sample is indicative of an acute bacterial infection in said individual, said device comprising
a) at least one surface on which a composition comprising:
   i) a proteoaminoglycan or dextran sulphate and ii) toluidine blue has been immobilized,
b) optionally a solid support onto which said at least one surface may be attached,
   wherein said at least one surface comprises a material suitable for immobilization of said composition comprising proteoaminoglycan or dextran sulphate and toluidine blue, and
   wherein on at least one surface of a) the ratio of the amount of a proteoaminoglycan or dextran sulphate to the amount of toluidine blue in said composition on said at least one surface is selected from the group of ratios consisting of:
      - about 100:1, such as about 90:1 to about 110:1 (surface 1);
      - about 140:1, such as about 135:1 to about 145:1 (surface 2);
      - about 165:1, such as about 160:1 to about 170:1 (surface 3);
      - about 180:1, such as about 175:1 to about 185:1 (surface 4);
      - about 200:1, such as about 190:1 to about 210:1 (surface 5); and
      - about 225:1, such as about 220:1 to about 230:1 (surface 6).

More particularly, the composition comprises dextran sulphate.

The ratio of the amount of proteoaminoglycan or dextran sulphate to the amount of toluidine blue in said composition is sufficient to detect the presence or absence of one or more acute phase protein(s) in said biological sample. Examples thereof are presented herein by specific predetermined ratios of a proteoaminoglycan or dextran sulphate to toluidine blue on a surface as examples of parameters sufficient to detect the presence of one or more acute phase protein(s) in a biological sample.

The different combinations of surfaces presented herein are particularly useful for detecting acute phase proteins in different biological fluids due to the composition of the acute phase proteins present in the various biological fluids. This may be due to the different elimination patterns of the acute phase proteins in the respective biological fluids. Therefore, different combinations of surfaces are presented herein.

The surface of a device according to the present disclosure can also be described as an "interacting" surface as it is capable of interacting with and binding a composition comprising toluidine blue and proteoaminoglycan.

A quantitative determination is possible by studying the colour of the at least one surface of the device after the biological sample has been added thereto. The stronger the blue colour, the more acute phase proteins are present in the biological sample. If the colour of all surfaces remains red or red-purple (horizon red), this means that there are no acute phase proteins in the sample.

Again, of course the "absence" of acute phase proteins in the biological sample shall not be construed as an absolute non-presence of acute phase proteins in said sample but rather at such a low level that there is essentially no acute phase proteins, or severe bacterial infection, present in said sample or said individual.

There is also provided herein, a kit of parts, said kit of parts comprising a device as disclosed herein, said kit further comprising a colour detecting means for quantitative determination of acute phase protein(s) detected by said device, and optionally instructions for use. Said kit of parts may comprise one, two or three (interacting) surfaces present on strip or said surface may be a tissue or a suitable material.

There is furthermore provided by the present disclosure, an *in vitro* method for quantitatively determining the presence or absence of one or more acute phase protein(s) in a biological sample from an individual, said presence of one or more acute phase protein(s) being indicative of an acute bacterial infection in said individual, said method comprising the steps of:
a) adding said biological sample to a device as provided herein,
b) incubating said biological sample on the surface of said device in step a), and thereafter
c) comparing the colour of the surface with a reference obtained with known amounts of acute-phase proteins to determine the presence or absence of one or more acute phase protein(s) in said biological sample.

The method is quantitative and fast and can instantly detect an increase or a decrease in a bacterial infection in an individual which makes it possible to continuously monitor an ongoing therapy, such as an antibiotic therapy.

In addition, there is provided herein a method for determining a suitable material for a surface as defined for a device herein, said method comprising the steps of:
a) soaking a material in a liquid solution comprising a composition comprising a proteoaminoglycan or dextran sulphate, and toluidine blue in any one of the ratios as defined herein,
b) determining the colour of the material after soaking in the solution of step a),
   wherein if the material presents the colour of toluidine blue after soaking, said material is suitable for a surface as defined herein, wherein the material will turn purple red or red when toluidine blue is bound to a proteoaminoglycan and/or dextran sulphate on said suitable material.

This introduces a simple method in order to choose which material is most appropriate to be used to immobilize a solution composed of proper amounts of proteoaminoglycan or dextran sulphate and Toluidine blue (interacting surface) in order to choose the most affordable one, and which material is most proper to be used as the supporting material for an (interacting) surface, in order to choose the most stable test configuration.

Various other objects and advantages of the present invention will become apparent from the drawings and the following description of the invention.

### BRIEF DESCRIPTION OF THE DRAWINGS

The following detailed description will be more fully understood in view of the figures, in which:

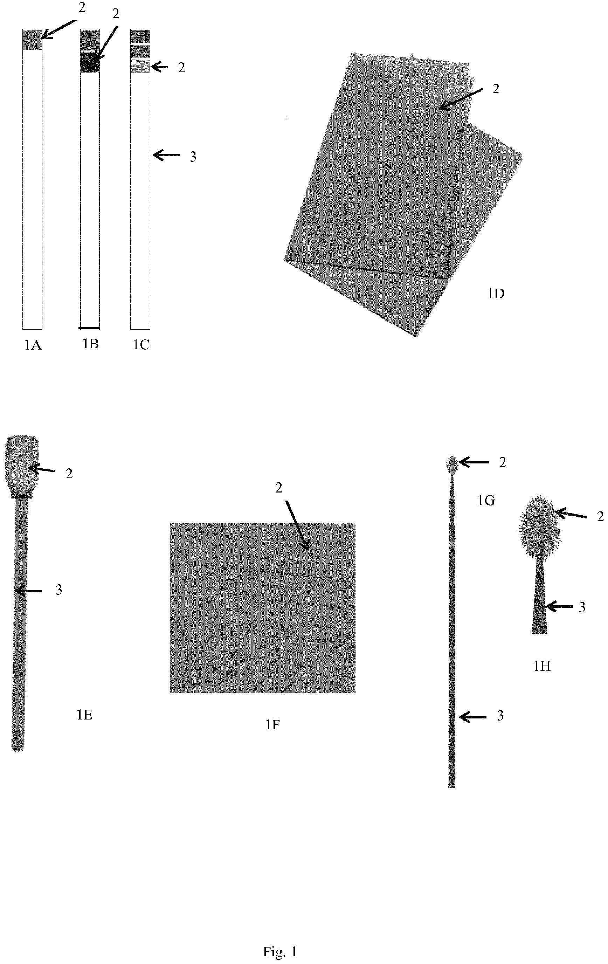

**Figure 1** describes examples of a device according to the disclosure, in the form of a strip with one surface (1A), a strip with two surfaces (1B), a strip with three surfaces (1C), a tissue comprising a surface (ID), a stick with a tissue comprising a surface (IE), the protein binding surfaces of the device in 1D (IF), a periodontal device comprising a surface (1G), and the protein binding surfaces of the device in 1G (1H).

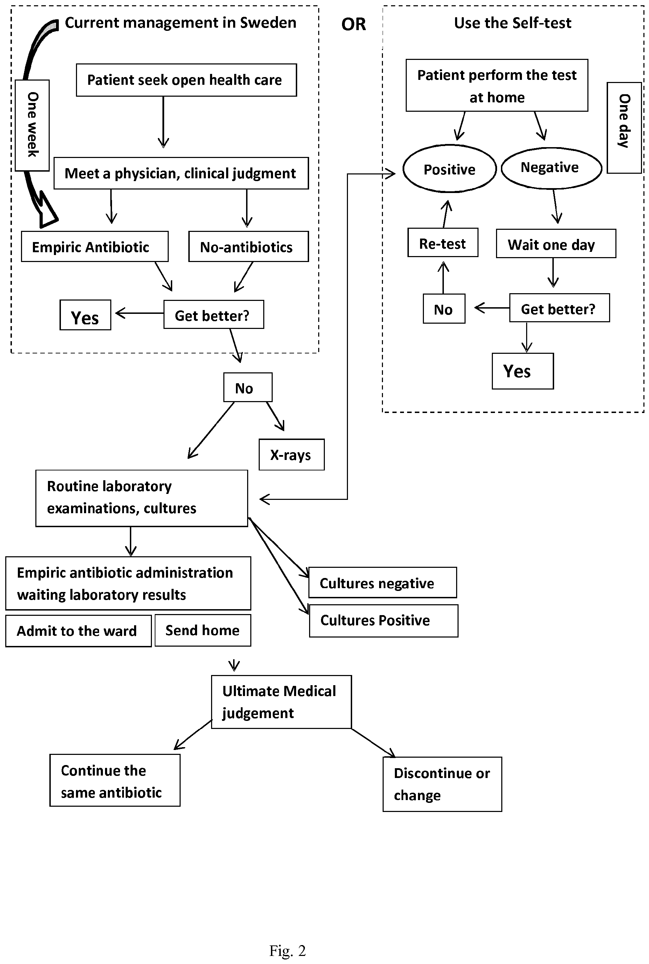

**Figure 2** describes a case management of acute respiratory tract infection with and without self-test. The Flow chart shows the patient management for respiratory infection in Sweden with and without the Index test (a device according to the present disclosure). It illustrates the improvement provided with a device and method according to the present disclosure as compared to the tests used today.

### DETAILED DESCRIPTION OF THE INVENTION

Recognizing the clinical importance and differences between recommended therapies, differential diagnosis between inflammatory disorders in the body has been the subject of several investigations. One major clinical problem is determining whether infection or other inflammatory disorders cause the disease. There are several markers that typically are used by physician to establish the right diagnosis such as microscopic analysis and culture of body fluids, white blood cell count, C-reactive protein, plasma procalcitonin and lactate. However, there are still no golden standards to be used. Problems in establishing correct diagnosis occurs daily while treating inflammatory disorders in bowel, ulcers, joint diseases, CNS disorders, peritoneal, pleural and pericardial effusions, among others. The amounts of routine markers such as CRP and WBC might be high in several disorders and cultures are not always positive in spite of an infection.

The innate immune system, also known as the non-specific immune system is an important subsystem of the overall immune system that comprises the cells and mechanisms that defend the host from infection. Neutrophils are the primary white blood cells that respond to a bacterial infection, so the most common cause of neutrophilia is a bacterial infection, especially pyogenic infections. On the other hand, acute viral infections cause lymphocytosis. Local acute phase proteins and regenerative factors are produced by neutrophils but not lymphocytes. Therefore detection of acute phase proteins in body fluids indicates a bacterial infection. The most important proteins produced during bacterial infection show high affinity to heparan sulphate proteoglycan (HSPG) in the extracellular matrix, as well as sulphated glycan such as dextran sulphate.

We have used this knowledge to produce a test comprising a device described herein resembling the affinity to extracellular matrix of acute phase proteins produced by neutrophils during bacterial infection. Hence, the present disclosure is related to a rapid method and device to visualize this phenomenon. We have established and herein describe a method based on inverted methachromacy. Addition of a proteoaminoglycan and/or dextran sulphate, to Toluidine blue causes change of blue colour to red. Addition of proteins with high affinity to proteoaminoglycan and/or dextran sulphate, (fast binding with minimal amounts) causes the red colour to change back to blue proportional to
a- The protein concentration
b- The binding affinity to dextran sulphate.

Thus the test results are highly correlated to presence of neutrophils in body fluids during bacterial infection detected by light microscopy and widely used since decades to detect infection world-wide. The test is intended to assess the presence of acute phase proteins produced by neutrophils e.g. in body fluids during infections.

The diagnostic value of detection of acute phase protein in body fluids using methachromacy to detect the protein in body fluid has been presented before (WO2010151222). However, these findings have now been further developed. Accordingly, there is presented herein:
1- The high correlation found between binding affinity of acute phase protein to a proteoaminoglycan or dextran sulphate, and number of neutrophils detected in body fluids during infection.
2- Description of important moment in methachromacy; Proportion between the amounts of proteoaminoglycan and/or dextran sulphate, to Toluidine blue in order to detect increasing amount of acute phase protein using inverted methachromacy.
3- The possibility to detect bacterial infection and to monitor the antibiotic therapy with high negative predictive value >95%.
4- Using this knowledge to rule out cases in which antibiotic therapy is of no value.

There is presented herein a suitable proportion of proteoaminoglycan, or dextran sulphate, and Toluidine blue in a solution that can detect different amounts of acute phase protein such as HGF. Examples thereof are shown further in the below (Table 1). Notably in this regard, methylene blue may also be used in a device or method herein as a substitute for Toluidine blue, even though it is preferred to use Toluidine blue in the context of the present disclosure. It is also envisaged that other substitutes for Toluidine blue may be used in the present context if such substitutes are evident to the skilled person.

Accordingly, it was shown herein to be particularly useful to use the approach of inverted metachromasy for detecting the presence of acute phase proteins in a biological sample of an individual. By adding a liquid solution comprising a composition comprising a proteoaminoglycan or dextran sulphate, and toluidine blue to a surface of a device, the proteoaminoglycan or dextran sulphate binding to toluidine blue is immobilized on the surface before the biological sample is added thereto. By adding the biological sample after the addition of toluidine blue to a proteoaminoglycan or dextran sulphate detection of acute phase proteins was shown to be possible in a more rapid and efficient manner than previously. Now, detection of acute phase proteins can be performed already about 1 minute after the biological sample has been added to the surface comprising a proteoaminoglycan or dextran sulphate and toluidine blue. It was also shown particularly efficient to immobilize a proteoaminoglycan or dextran sulphate and toluidine blue (initially present in an aqueous solution added to said surface) on a surface before the biological sample is added thereto.

An example of liquid solutions comprising compositions comprising a proteoaminoglycan or dextran sulphate and toluidine blue is described in Table 1. The different solutions used in diagnosis of infection in different body fluids in shown in Table 2. Dextran sulphate is provided as an example. Hence, ratios in about the below amounts are very useful in a method or device herein as further explained in the below.

**Table 1: Proportions to detect different amounts of acute phase protein.**

| Concentration gradient (surface no.) | Dextran sulphate H₂O₄S ▪ xNa ▪ x, CAS 9011-18-1 MW 500000 (g/mol) | Toluidine Blue CAS 92-31-09 C15H16N3S, MW 270.37 (g/mol) | Quotation |
|---|---|---|---|
| 1 | 1.5g/500ml MQ | 150ml 100mg/L | 100 |
| 2 | 3.36g/500ml | 250ml 100mg/L | 137.75 |
| 3 | 5.75g/500ml | 350ml 100mg/L | 164.8 |
| 4 | 10.6g/500ml | 600ml 100mg/L | 180 |
| 5 | 15.86g/500ml | 800ml 100mg/L | 200 |
| 6 | 27g/500ml | 1200ml 100mg/L | 226 |

**Table 2: The recommended combinations when making different test strips for different body fluids or samples**

| **Sample** | **included surfaces on strip** |
|---|---|
| Recombinant HGF | 1+2 |
| Sputum professional test | 3+4+5 |
| Sputum self-test | 4 |
| Urine professional | 3+5 |
| Urine self-test | 4 |
| Cerebrospinal fluid | 3+4 |
| Ulcer secretion | 5+6 |

Based on the knowledge that HGF is one of the proteins produced at the acute phase of bacterial infection and captured by Dextran sulphate, we have shown that the results from the (index) strip test were significantly correlated with the results from the antibodies-based ELISA test for HGF (n=47; Pearson's Correlation, R=0.71; P<0.0001) (Table 3).

**Table 3: The HGF concentration captured by the dextran sulfate immobilized in different concentrations on the strip. The Elisa kit had a detection rate of 0.04 ng/ml-8. 0 ng/ml**

| **Test results in strip with 3 surfaces and different concentrations** | **concentration ng/ml Range (Median)** |
|---|---|
| **Negative** | 0.04- 8.34 (0.67) |
| **Positive surface 1** | 0.04-7.92 (2.87) |
| **Positive surface 1+2** | 2.71 - 8.31 (8.08) |
| **Positive surfaces 1+2+3** | 0.30- 8.77 (8.08) |

### The material used in preparation of the detecting/interacting surfaces and supporting material (solid support):

In order to immobilize the solutions containing dextran sulphate and Toluidine blue, different materials may be used. As a suggestion, Relia Flow 800 Ahlström is a good material that does not react to low or high pH or to high amounts of hemoglobin. However, in order to decrease the costs and during epidemics it is possible to test the materials and choose the one at the lowest price that still experiences the appropriate characteristics.

Any material that after soaking in the solution keeps the purple red colour of solution, when dried, might be used for detecting surface. As an example Geotextile is a cheap and appropriate material.

For the support of the strip test, we use a material that does not bind to dextran sulfate in order to increase the stability of the test. We can test materials by soaking in a solution containing a proteoaminoglycan, dextran sulphate and Toluidine blue. The material is identified as appropriate for the test if it takes the blue colour. The natural products such as wood, paper, wool, cotton or silk do not bind to dextran sulphate and can be used as supporting (i.e. as a solid support) in the device herein (strip test).

### The Inverted Methachromacy:

Methylene blue (O-Toluidine) is an excellent metachromatic dye that changes from blue to pink upon binding to high-molecular-weight polysaccharides, such as sulfated glycan [Pham NA, 2007]. The pink dye will then quickly turn back to blue following addition of a proportional amount of a protein with high affinity to sulfated glycan (inverted methachromacy).

This approach has now been used to develop a new strip test (device) to assess the presence of proteoaminoglycan (and/or dextran sulphate)-binding proteins in sputum. Other biological samples may also be used in the context of the present disclosure.

Without wishing to be bound by theory, a theoretical support for the presently studied approach to detect presence of acute phase protein in body fluids during infection is suggested to be as follows. (1) Glucosaminoglycan (GAG) polymer chain with negative charges attaches to glucosaminoglycan binding material. (2) Positive toluidine blue (TBO) molecules attach to the polymer chain of the GAG, concentrate, and form stacks, and the color changes from blue to red. (3) Sample containing acute phase protein is added, and the protein binds to GAG. (4) TBO molecule stacks are destroyed, becoming free dissolved TBO molecules, and the color changes from red to blue. Hence, when the blue colour is detected, this means that there is a presence of acute phase proteins in said biological sample that is tested.

Accordingly, there is provided herein a device for quantitatively determining the presence or absence of one or more acute phase protein(s), such as Hepatocyte Growth Factor (HGF), in a biological sample from an individual, a presence of one or more acute phase protein(s) in said biological sample being indicative of an acute bacterial infection in said individual, said device comprising
a) at least one surface on which a composition comprising a i) proteoaminoglycan or dextran sulphate and ii) toluidine blue has been immobilized,
b) optionally a solid support onto which said at least one surface is attached,
   wherein said at least one surface comprises a material suitable for immobilization of said composition comprising proteoaminoglycan or dextran sulphate and toluidine blue; and
   wherein on at least one surface of a) the ratio of the amount of a proteoaminoglycan or dextran sulphate to the amount of toluidine blue in said composition on said at least one surface is selected from the group of ratios consisting of:
      - about 100:1, such as about 90:1 to about 110:1 (surface 1);
      - about 140:1, such as about 135:1 to about 145:1 (surface 2);
      - about 165:1, such as about 160:1 to about 170:1 (surface 3);
      - about 180:1, such as about 175:1 to about 185:1 (surface 4);
      - about 200:1, such as about 190:1 to about 210:1 (surface 5); and
      - about 225:1, such as about 220:1 to about 230:1 (surface 6).

The ratio of the amount of proteoaminoglycan or dextran sulphate to the amount of toluidine blue in said composition is sufficient to detect the presence or absence of one or more acute phase protein(s) in said biological sample. Examples thereof are presented herein.

Said biological sample usually comprises a biological fluid such as e.g. sputum, urine or the like or as further described herein.

Said device may comprise at least two surfaces, such as three surfaces, on which a composition comprising a proteoaminoglycan or dextran sulphate and toluidine blue has been immobilized, wherein the ratio of the amount of a proteoaminoglycan or dextran sulphate to the amount of toluidine blue in the composition on said first surface is different than the ratio of the amount of proteoaminoglycan or dextran sulphate to the amount of toluidine blue in the composition on said second surface and/or optionally further different from said third surface.

Accordingly, there is provided herein a device comprising at least one surface, wherein the ratio of the amount of a proteoaminoglycan or dextran sulphate to the amount of toluidine blue in said composition present on said at least one surface is:
- about 100:1, such as about 90:1 to about 110:1 (surface 1);
- about 140:1, such as about 135:1 to about 145:1 (surface 2);
- about 165:1, such as about 160:1 to about 170:1 (surface 3);
- about 180:1, such as about 175:1 to about 185:1 (surface 4);
- about 200:1, such as about 190:1 to about 210:1 (surface 5); or
- about 225:1, such as about 220:1 to about 230:1 (surface 6).

More particularly, said ratio for surface 1 may be about 100:1, for surface 2 may be about 138:1, for surface 3 may be about 165:1, for surface 4 may be about 180:1, for surface 5 may be about 200:1 and/or said ratio for surface 6 may be about 226:1.

There is provided a device herein comprising any combination of one or more these surfaces(s).

A device provided herein may comprise one or more of the above mentioned surfaces (ref. no. 2 in Figures 1A-1H), wherein the surfaces are attached to or part of a device for determining the presence of one or more acute phase proteins. The surfaces, if more than one, may be presented on the device spaced apart, so as to allow the different surfaces to use different ratios of the amounts of proteoaminoglycan or dextran sulphate and toluidine blue. This is exemplified e.g. in Fig. 1B and 1C. Examples of a device according to this disclosure are shown in Figures 1A-1H. Hence, a device may be in the form of a strip (1A-1C), a tissue (ID, IF), a stick comprising a surface (IE), a stick adapted for dental applications with a brush-like surface (1G, 1H) or the like. The material used for the surface is a material that allows for the immobilization of a proteoaminoglycan or dextran sulphate and toluidine blue on said surface. If said surface is placed, or attached onto a solid support (ref.no. 3 in Fig. 1), this solid support is usually manufactured in a material that will not bind a proteoaminoglycan or dextran sulphate and toluidine blue, as further described herein. The device according to the present disclosure may also be introduced as a part of a diaper adapted for a baby or for an adult. The device may be arranged in the diaper in the form of a strip preferably in the crotch of the diaper, said strip comprising one or more surfaces as disclosed herein making it possible to continuously monitor any bacterial content in the urine or other bodily fluid. This could facilitate early detection of a bacterial infection in the urinary or genital tract, such as a urinary infection. A colour change, as previously explained herein, indicating the presence of a bacterial infection can be visualized when wearing the diaper. Accordingly, there is also provided herein a device, wherein said device is present in a diaper for detection of an acute bacterial infection.

It should be noted that the above mentioned ratios may also vary slightly. The ratios between the amounts of proteoaminoglycan or dextran sulphate, and toluidine blue are shown herein to present a particularly useful composition for detecting the presence of acute phase proteins in a biological sample.

The amounts of proteoaminoglycan or dextran sulphate and toluidine blue are amounts in weight of proteoaminoglycan or dextran sulphate and toluidine blue, of dry material that has been added to an aqueous liquid. As previously mentioned herein, firstly, toluidine blue may be added to MQ water and secondly, proteoaminoglycan, and/or dextran sulphate, may be added to MQ, and thereafter, these two aqueous solutions are mixed.

Furthermore, there is provided herein a device, wherein said device comprises two or more, such as two, three, or four of the surfaces: surface 1, surface 2, surface 3, surface 4, surface 5, and surface 6. Such more than one surface(s) are usually presented on a test strip.

In addition, there is also provided a device comprising surface 1 and surface 2. A device comprising one or more of surface 1 and surface 2 may be used for detecting recombinant HGF. In addition, there is also provided a device comprising at least one, such as two or three of: surface 3, surface 4 and/or surface 5. A device comprising surface 3, surface 4 and/or surface 5 is particularly useful for determining the presence of acute phase proteins in biological samples comprising sputum, broncho-alveolar lavage, a nasal secretion, cerebrospinal fluid, urine or renal secrete. A device comprising surface 3 and/or surface 4 is useful for determining the presence of acute phase proteins in a biological sample comprising joint fluid. This may be used in determining the presence of bacterial osteoarthritis. A device comprising surface 3 is particularly useful for determining the presence of acute phase proteins in saliva and/or periodontal secretions. This is also illustrated in Table 2.

Furthermore, there is also provided a device comprising surface 5 and surface 6. A device comprising surface 5 and/or surface 6 is particularly useful for determining the presence of acute phase proteins in biological samples comprising ulcer secretion.

In a device according to the present disclosure, said proteoaminoglycan may be selected from the group consisting of HSPG (Heparan Sulphate Proteoglycan), and heparin sulphate. More particularly, said composition may comprise dextran sulphate.

There is also provided herein, a kit of parts, said kit of parts comprising a device as disclosed herein, said kit further comprising a colour detecting means for quantitative determination of acute phase protein(s) detected by said device, and optionally instructions for use. Said colour detection means may use spectrophotometry. In addition, said colour detecting means may be any means, device or the like, that is suitable for determining the colour of the at least one surface used in a device according to the present disclosure. It can be a device capable of automatically determining the colour, or it can be performed by using the mere eye and a reference chart with different colours (i.e. a colour chart) indicating the amount of acute phase proteins present in the sample for use in comparing with the results of the test.

The device according to the present disclosure can also be presented in a detector of choice for interpreting the results of the method as presented herein. For example, a hand-held detector for convenient use, such as in the form of a pen (pen-shaped detector) for rapid indication of a bacterial infection in a sample is envisaged herein, said detector providing for positioning of the device within the detector. The detector may have a replaceable single-use tip containing a transparent version of the detection reagent. Introduction of sample into the tip, may e.g. be passive (the sample enters the reactive area in the tip when in contact with the sample solution) or active (the detector includes a device for actively drawing sample solution into the tip). The detector may have integrated optics and electronics enabling a sensitive detection of the color change in the reaction area of the tip. The detector may also include a display indicating the result and optionally an ON/OFF button. The design/geometrical position of the light guides and the light detector will most likely be dependent of the chosen design of the tip. Accordingly, there is also provided herein a device, wherein said device is present in a hand-held device for detection of an acute bacterial infection.

There is furthermore provided by the present disclosure, an *in vitro* method for quantitatively determining the presence or absence of one or more acute phase protein(s) in a biological sample from an individual, said presence of one or more acute phase protein(s) being indicative of an acute bacterial infection in said individual, said method comprising the steps of:
a) adding said biological sample to a device as provided herein,
b) incubating said biological sample on the surface of said device in step a), and thereafter
c) comparing the colour of the surface with a reference obtained with known amounts of acute-phase proteins to determine the presence or absence of one or more acute phase protein(s) in said biological sample.

The *in vitro* method may be performed in a manner wherein step c) is performed directly after step b), or at least within about 2 minutes, such as about 1 minute. This timeframe illustrates the efficacy and rapid nature of the method and device presented herein.

Hence, said method allows diagnosing a bacterial infection in an individual, and comprises contacting a body fluid sample from the individual with a means, e.g. a colorimetric device to determine relative levels of acute phase proteins by neutrophil's production stimulated by bacterial infection in the affected organ, and correlating the determined levels to an acute infection. This provides a method for rapid determination of amounts of acute phase proteins in the body fluids (biological samples), such as, for example, urine, cerebrospinal fluid, exhaled breath condensate, broncho-alveolar lavage, nasal secretion, renal secrete, periodontal secretions, sputum, semen, saliva, joint fluid and ulcer secretion. As mentioned herein, knowledge of the concentration of acute phase protein facilitates diagnosis and monitoring of disease.

The acute phase protein determined in said *in vitro* method may be Hepatocyte Growth Factor (HGF), a derivative thereof or a protein related thereto. Said acute phase protein may also be calprotectin.

Said method employs an inverted methachromic method for visualization of the binding between toluidine blue and proteoaminoglycan. A positive immunological reaction is indicative of the presence of acute phase protein, e.g. HGF (both active and inactive forms) in the sample. A positive immunological reaction provides a blue surface (toluidine blue has been released from the proteoaminoglycan).

There is provided herein an efficient proportion (ratio) between a proteoaminoglycan 2. (or dextran sulphate) and Toluidine blue that can detect different amounts of acute phase protein, such as HGF or calprotectin. This is illustrated by the respective surfaces presented herein. Hence, it is shown herein that the efficient proportion of proteoaminoglycan or dextran sulphate and Toluidine blue results in a solution that by immobilization on proper filter can diagnose grade of bacterial infection.

There is also provided an *in vitro* method, wherein said device comprises surface 1 and surface 2 as defined herein. Said device used in said *in vitro* method may also comprise at least one, such as one, two or three of: surface 3, surface 4 and surface 5 as defined herein. When said surfaces are used in said device, said biological sample added to said device particularly comprises sputum, broncho-alveolar lavage, a nasal secretion, cerebrospinal fluid, urine or renal secrete. There is also provided an *in vitro* method, wherein said device comprises surface 5 and surface 6, as defined herein and wherein said biological sample particularly comprises ulcer secretion.

Thus, herein it is shown that certain proportions (ratios) of amounts of proteoaminoglycan and/or dextran sulphate in relation to toluidine blue are particularly useful for determining the presence or absence of acute phase proteins in biological samples, and even more, certain ratios are particularly suitable for biological samples of a particular type (e.g. sputum or urine sample). This is illustrated in Table 2. For example, for sputum and for a self-test at home, it is useful with a device comprising surface 4. For professional use, said device still comprises surface 4, but it also comprises surface 3 and surface 5 for even more sensitive results. The optimization of these characteristics presents an even more efficient way of determining the amounts of acute phase proteins in a biological sample, and thereby of following infection progression and/or (antibiotic) therapy.

Accordingly, the present disclosure in broader terms relates to diagnosis of bacterial respiratory infection; community acquired or nosocomial, from viral or chronic respiratory disorder by analysis of sputum, broncho-alveolar lavage or nasal secretion as further defined herein. An appropriate (interacting) surface for these particular biological samples is presented herein (surface 3, surface 4, and/or surface 5).

There is also provided a use of a device and a method presented herein in the diagnosis of proximal urinary tract infection in need of wide-range and long antibiotic therapy from distal urinary tract infection or no infection, by analysis of urine or renal secretion as further defined herein. An appropriate (interacting) surface for these particular biological samples is presented herein (surface 3, surface 4, and/or surface 5).

There is also provided a use of a device and a method in the diagnosis of bacterial meningitis; community-acquired or nosocomial, from other causes of pleocytosis, by analysis of cerebrospinal fluid as further defined herein. An appropriate (interacting) surface for these particular biological samples is presented herein (surface 3 and/or surface 4).

There is also provided a use of a device and a method in the diagnosis of acute bacterial ulcer infection by analysis of ulcer secretion as further defined herein. An appropriate (interacting) surface for this particular biological sample is presented herein (surface 5 and/or surface 6).

In addition, there is also provided a use of a device and a method in the diagnosis of bacterial osteoarthritis by analysis of joint fluid. An appropriate (interacting) surface for this particular biological sample is presented herein (surface 3 and/or surface 4).

There is also provided the use of a device and a method for determining of periodontal status by analysis of saliva or periodontal secretions. An appropriate (interacting) surface for this particular biological sample is presented herein (surface 3 or surface 4).

There is also provided an *in vitro* method as defined herein, wherein determining the presence or absence of one or more acute phase protein(s) as explained herein is used for monitoring a therapy, such as antibiotic therapy in an individual. In such a method, the concentration of acute phase proteins may be monitored and quantitatively rapidly determined and with regular intervals and it may efficiently be determined e.g. if the wrong therapy is being used for a certain individual, or how efficient the therapy is. This e.g. avoids unnecessary use of antibiotics, when the antibiotics used may not be appropriate for the individual/particular infection.

It is also shown herein that an efficient proportion of proteoaminoglycan and/or dextran sulphate and Toluidine blue presents a solution that by immobilization on proper filter can diagnose grade of bacterial infection and/or monitor an antibiotic therapy. It is shown herein the value of a test based on a described method to rule out cases of non-bacterial infection and thereby reduce antibiotic consumption.

In a device according to the present disclosure, a material for the at least one surface for detecting acute phase proteins needs to be selected, based on properties that it will be suitable for the purpose. This is further described herein.

Accordingly, in addition, there is provided herein a method for determining a suitable material for a surface as defined for a device herein, said method comprising the steps of:
a) soaking a material in a liquid solution comprising a composition comprising a proteoaminoglycan or dextran sulphate, and toluidine blue in any one of the ratios as defined herein,
b) determining the colour of the material after soaking in the solution of step a),
   wherein if the material substantially presents the colour of toluidine blue bound to proteoaminoglycan or dextran sulphate after soaking, said material is suitable for a surface as defined herein, wherein the material will turn purple red or red when toluidine blue is bound to a proteoaminoglycan and/or dextran sulphate on said suitable material. Furthermore, examples of suitable materials for the surface are: e.g. geotextile or filters comprising polyester fibers.

The invention is now further described by the following examples. The examples are illustrative and should not be construed as limiting the scope of the invention which is defined by the appended claims.

### EXPERIMENTAL SECTION

### Example 1:

### Clinical Performance study of Dexact- Resp (a device of the present disclosure) on left-over sputum in diagnosis of bacterial pneumonia

**Background:** In patients with clinical symptoms of respiratory infection, rapid identification of cases requiring antibiotic therapy is crucial to avoid development of multiple resistant bacteria. Identification of local acute-phase reactants can help assess the host's response to bacterial infection at the injury site. Here we developed an affordable, stable, feasible, and accurate diagnostic tool based on a locally produced protein with specific binding affinity to polysaccharides. We further evaluated the ability of the novel test strip to detect bacterial infection in cases of pneumonia.

### METHODS

A strip test (the so called index test) was developed based on inverted metachromacy. Leftover sputum samples (n=467) from patients with suspected pneumonia were blindly tested using the index test (Table 4). These results were compared to the ultimate outcomes determined based on independent clinical and laboratory assessments performed by the patient's physician.

### RESULTS

The index test showed a 98.9% sensitivity and 90.2% specificity for detecting the host reaction to high levels of pathogenic bacteria within 2 minutes (Table 5). The golden standard used was sputum culture and PCR. However, the sensitivity of standard tests such as sputum cultures, PCR, antigen detection and lung X-ray to detect bacterial pneumonia (judged and treated by physician in charge) was <70%.

Examples of devices useful for this test are presented in Fig. 1.

### CONCLUSIONS

The novel screening test based on inverted methachromacy has the potential to reduce mortality and morbidity in cases of pneumonia, to guide antibiotic prescription, and to conserve resources, which is especially vital in poorly equipped centers and rural areas (Fig. 2).

**Table 4. Summary of the study material and index test results. n denotes number of cases, N denotes number of patients.**

| **Leftover sputum collected at University Hospital in Linköping Sweden and freshly analyzed (n=467, N=344).** | **Index test strip negative/positive (n=on antibiotics)** | **% Highly positive index test strip results** | **Sputum positive by culture or PCR** | **CRP> 20** |
|---|---|---|---|---|
| ***Judged as respiratory infection by the physician in charge*** | | | | |
| Bacterial respiratory tract infection total (n=134; N=95) Pathogenic bacteria (n=97) Subgroup a | 13/121 (n=8) 1/96 | 95/134=70% | 134, including 19 *M. catarrhalis, 32 HI, 12 S. aureus,* 25 *P. aeruginosa, 21 Pnc, 9 Mycoplasma, 9 Enterobacteriac eae,* etc. | 40 |
| Viral respiratory infection (n=6, N=5) Subgroup b | 3/3 (n=1) | 3/6=50% | 3 H1N1, 3 Influenza A | 6 |
| Fungal respiratory infection (n=19, N=12) Subgroup c | 10/9 (n=5) | 7/19=36% | 19 | 0 |
| Culture-negative infections with x-ray manifestations (n=36, N=28) Subgroup d-1 | 8/28 (n=25) | 12/36=33% | 0 | 30 |
| Etiologically non-verified respiratory infections (n=78, N=61) Subgroup d-2 | 12/66 (n=18) | 24/78=30% | 0 | 21 |

| ***Judged as non-infectious lung manifestation by the physician in charge*** | | | | |
|---|---|---|---|---|
| Respiratory infections controlled at convalescence (n=69, N=48) Subgroup e | 65/4 (n=2) | 0 | 0 | 5 |
| Colonization of | 28/1 | 0 | 29 | 4 |
| bacteria in respiratory tract (n=29, N=20) Subgroup f | (n=1) | | | |
| Culture-negative lung manifestation during non-infectious diseases (n=96, N=78) Subgroup g | 82/14, including 18 respiratory insufficiencies, 13 asthmas, 15 chronic obstructive lung diseases, 9 lung stasis, 8 lung fibrosis, 13 lung tumor, and 11 autoimmune diseases | 6/96=6% | 0 | 18 |

**Table 5: The index test results**

| ***Strip test result*** | ***Severe bacterial infection*** | ***No infection*** | ***Total*** |
|---|---|---|---|
| Positive | 96 | 19 | 115 |
| Negative | 1 | 175 | 176 |
| Total | 97 | 194 | 291 |

Table 5: The test evaluation was performed between groups: Positive control: Invasive bacterial infection caused by *Streptococcus pneumonia, Pseudomonas aeruginosa, Hemophylus influenza, Streptococci, Mycoplasma pneumonia, Staphylococcus aureus,* Negative control: Infection was ruled-out. Sensitivity was 98.97, Specificity was 90.21, Positive predictive value was 83.48 and Negative Predictive value was 99.43.

**Table 6: The table shows the results from the standard test performed by the hospital.**

| Total diagnostic tests and/or X-ray manifestation | Ultimate diagnosis: Respiratory infection (bacterial, fungal, viral) | Ultimate diagnosis: NO INFECTION | Total |
|---|---|---|---|
| Positive | 185 | 29 | 214 |
| Negative | 88 | 165 | 253 |
| Total | 273 | 194 | 467 |

The standard diagnostic procedures inclusive cultures, PCR, antigen detection and X-ray were performed on the patients, in which the left-over sputum was collected (n467) and used for evaluation of the strip test (Table 6). The ultimate diagnosis was set by the physician in charge after medical evaluation on basis of standard tests and clinical signs and symptoms of patient, without knowledge about the result from the strip test. The test performance for the standard diagnostic tests was 0.68 sensitivity, 0.85 specificity, 0.86 positive predictive value and 0.65 negative predictive value.

### Example 2:

### Clinical Performance study of Dexact-Urine on left-over urine in diagnosis of urinary tract infection

The urine strip test could differentiate between cases of severe proximal urinary tract infection from uncomplicated distal urinary tract infection and healthy (Table 7).

**Table 7: Clinical performance study of Dexact urine**

| ***Left-over urine collected at University Hospital in Linköping Sweden 29^{th} April-9^{th} may 2016 (1-97 years, median 64 year) and freshly analyzed (n=538)*** | *D****exact strip negative (1 surface positive)* /*both surfaces positive*** | ***Percent Dexact positive in both surfaces that indicates serious cases (sensitivity or specificity)*** | ***Culture urine positive*** | ***CRP** >20* |
|---|---|---|---|---|
| Abdominal disorder n=32 | 15(16)/1 | 3% | 8 | 7 |
| Control of urine during brain injuries N=10 | 3+7/0 | 0 | 3 | 0 |
| Renal calculus uncomplicated n=8 | 2(6)/0 | 0 | 2 | 0 |
| Complicated infections n=12 | 3+3/6 | 50% | 4 | 9 |
| Control urine as a part of investigation n=109 | 65 (38)/6 | 5% | 11 | 0 |
| Distal UTI n=147 | 71(66)/10 | 6.8% (93% | 98 | 6 |
| | | specificity for exclusion of non-serious cases 71+66/147) | | |
| febrile UTI n=79 | 6 (28)/45 | 56% (92% sensitivity for positive results 45+28/79) | 56 | 45 |
| Fever of unknown origin n=29 | 11 (12)/6 | 20% | 6 | 18 |
| Hematuria n=6 | 3 (3)/0 | 0 | 3 | 1 |
| Infection in genitalia (vaginitis, prostatitis, epididymitis) n=21 | 7 (10)/4 | 19% | 2 | 3 |
| Urogenital malignancy n=16 | 8 (7)/1 | 6% | 1 | 2 |
| Control during Pregnancy n=18 | 10 (5)/3 | 16% | 5 | 0 |
| Respiratory infection n=21 | 10 (9)/2 | 9% | 6 | 16 |
| Septicemia n=10 | 1 (7)/2 | 20% | 7 | 10 |
| Urinary retention n=9 | 5 (3)/1 | 11% | 5 | 1 |
| Unknown Medical history n=12 | 1 (9)/2 | | 6 | 0 |
| Volunteers joined a health control campaign 2014-2016 (18-81 years) and analyzed twice, fresh and once thawed (N=69) | 63 (5)/1 | 1.4% (91% specificity for negative result 63/69) | 3 | 1 |

Table 7 shows the left-over samples included in the validation of Renal-ITIS test, divided in groups based on clinical diagnosis established by the medical teams at different departments at the region hospital in Linköping, Sweden in May 2016. The control group was enrolled 2014-2016. Mann-Whitney U-test : Significant difference in Elisa concentration between Renal-ITIS positive and negative samples p<0.001.

### Example 3:

### Clinical Performance study of Dexact- CSF on patients with nosocomial bacterial meningitis included at the Department of Neurosurgery

Currently, the diagnosis of bacterial meningitis remains based on standard methods of direct microscopy, differential analyses of white blood cells, lactate, and protein, and cultures of blood and CSF [Baty V, 2000]. However, post-neurosurgical infections are difficult to distinguish from the effects of neurosurgical procedures [Baty V, 2008; Walti LN, 2013]. For example, when patients develop fever within days after surgery, a determination of cells and lactate in the CSF cannot provide reliable information. Moreover, due to prophylaxis treatments, the cultures are negative in a large group of patients, and the presence of skin flora, like *Coagulase negative Staphylocococcus* or *Propionbacterium acnes,* may indicate either infection or contamination.

Study on cerebrospinal fluid from cases that were admitted to the Department of Neurosurgery, University in Linköping, Sweden has shown a 100% sensitivity and Negative Predictive value for the Strip test (Table 8).

**Table 8. Table shows the Clinical Performance of Strip test to distinguish bacterial meningitis in cerebrospinal fluid.**

| **CSF samples (n=81)** | **Verified post-neurosurgical maningitis** | **Negative CSF cultures and no definitive sign of infection** |
|---|---|---|
| Positive CSF-strip test | TP=21 | TN=51 |
| Negative CFS-strip test | FN=0 | FP=9 |
| Negative Predictive value 100% | Sensitivity 100% | Specificity 85% |

### References

**1-** Baty V, Viel JF, Schuhmacher H, Jaeger F, Canton P, Hoen B. Prospective validation of a diagnosis model as an aid to therapeutic decision-making in acute meningitis. Eur J Clin Microbiol Infect Dis. 2000;19(6):422-6
**2-** Bean N H and Griffin PM. Foodborne disease outbreaks in the United States, 1973-1987: Pathogens, vehicles, and trends. J Food Prot, 1990; 53:804-817.
**3-** Bergeron JA, Singer M, Metachromasy: an experimental and theoretical reevaluation. J Biophys Biochem Cytol 4 (1958) 433-457.
**4-** Bonelli RR; Moreira BM, Picão RC. Antimicrobial resistance among Enterobacteriaceae in South America: History, current dissemination status and associated socioeconomic factors. Drug Resistance Updates, 2014; 17:24-36
**5-** Cohen-Nahum K, Saidel-Odes L, Riesenberg K, Schlaeffer F, Borer A. Urinary tract infections caused by multi-drug resistant Proteus mirabilis: Risk factors and clinical outcomes. Infection. 2010 Feb;38(1):41-6.
**6-** Falagas ME, Karageorgopoulos DE. Extended-spectrum β-lactamase-producing organisms. Journal of Hospital Infection. 2009;73(4):345-354.
**7-** Gaieski DF, Mikkelsen ME, Band RA, Pines JM, Massone R, Furia FF; Shofer FS, Goyal M. Impact of time to antibiotics on survival in patients with severe sepsis or septic shock in whom early goal-directed therapy was initiated in the emergency department. Critical Care Medicine, 2010;48:1045-1053
**8-** Gopalakrishnan R, Sureshkumar D. Changing trends in antimicrobial susceptibility and hospital acquired infections over an 8 year period in a tertiary care hospital in relation to introduction of an infection control programme. J Assoc Physicians India. 2010; 58:25-31
**9-** Harris JC, DupontHL, RB. Fecal leukocytes in diarrheal illness. Ann Intern Med. 1972 May;76(5):697-703
**10-** Hirsch E, Tam VH. Impact of multidrug-resistant Pseudomonas aeruginosa infection on patient outcomes. Expert Rev Pharmacoecon Outcomes Res. 2010 Aug; 10(4): 441-451.
**11-**Infectious Diseases Society of America (IDSA). Combating antimicrobial resistance: Policy recommendations to save lives. Clinical Infectious Diseases, 2011;52(S5):S397-S428
**12-** Kassakian SZ; Mermel LA, Changing epidemiology of infections due to extended spectrum beta-lactamase producing bacteria. Antimicrobial Resistance and Infection Control 2014, 3:9
**13-**Kerremans JJ, Verboom P, Stijnen T, Hakkaart-van Roijen L, Goessens W, Verbrugh HA, Vos MC. Rapid identification and antimicrobial susceptibility testing reduce antibiotic use and accelerate pathogen-directed antibiotic use. Journal of Antimicrobial Chemotherapy, 2008;61, 428-435
**14-**Lam SW, Bauer SR, Duggal A. Procalcitonin-based algorithms to initiate or stop antibiotic therapy in critically ill patients: Is it time to rethink our strategy? International Journal of Antimicrobial Agents, 2015; Epub ahead of print
**15-**Laxminarayan R, Duse A, Wattal C, Zaidi A, Wertheim H, Sumpradit N, Vlieghe E, Hara GL, Gould I, Goossens H, Greko C, So AD, Bigdeli M, Tomson G, Woodhouse W, Ombaka E, Peralta A Q, Qamar F, Mir F, Kariuki S, Bhutta Z, Coates A, Bergstrom R, Wright GD, Brown E, Cars O, Antibiotic resistance-the need for global solutions. Lancet Infect Dis 2013;13: 1057-98.
**16-** Lönn J, S Nakka, H Olsson T Bengtsson S Almer F Nayeri. Differences in expression of hepatocyte growth factor in acute and chronic bowel inflammation-Implication for diagnosis? Advances in Bioscience and Biotechnology, 2013, 4:33-42.
**17-**Melendez JH, Frankel YM, An AT; Williams L, Price LB, Wang NY, Lazarus GS, Zenilman JM. Real-time PCR assays compared to culture-based approaches for identification of aerobic bacteria in chronic wounds. Clin Microbiol Infect, 2010; 16: 1762-1769.
**18-**Molnarfi N, Benkhoucha M, Funakoshi H, Nakamura T, Lalive PH. Hepatocyte growth factor: A regulator of inflammation and autoimmunity.Autoimmun Rev, 2015;14(4):293-303.
**19-**Nadanaka S, Kitagawa H. Heparan sulphate biosynthesis and disease. J Biochem, 2008 Jul;144(1):7-14.
**20-**Nakamura T, Sakai K, Nakamura T, Matsumoto K. Hepatocyte growth factor twenty years on: Much more than a growth factor. J Gastroenterol Hepatol, 2011; 26:188-202.
**21-**Nayeri F, Hagberg L, Brudin L, Roberg M, Söderström C, Forsberg P.A measurement of Hepatocyte growth factor values in CSF; a comparison between septic and non-septic meningitis. J.Infect.Dis, 2000; 181:2092-2094.
**22-**Nielsen, L., Kjerulf, A. and Arpi, M. (2015) Increasing Incidence of Extended-Spectrum Beta-Lactamase-Producing Enterobacteriaceae (ESBL) and the Relation to Consumption of Broad-Spectrum Antimicrobial Agents 2003-2011 in a Large Area of Copenhagen, Denmark. Open Journal of Medical Microbiology, 5, 28-42.
**23-** O'Ryan M, Lucero Y, O'Ryan-Soriano MA, Ashkenazi S.An update on management of severe acute infectious gastroenteritis in children. Expert Rev Anti Infect Ther. 2010;8(6):671-82.
**24-** Oliver A, Mulet X, Lopez-Causape C, Juan C, The increasing threat of Pseudomonas aeruginosa high-risk clones. Drug Resist Updat. 2015;21-22:41-59.
**25-** Ostholm-Balkhed A, Tärnberg M, Nilsson M,Nilsson LEHanberger H, Hällgren A. Travel-associated faecal colonization with ESBL-producing Enterobacteriaceae: incidence and risk factors. J Antimicrob Chemother 2013;68(9):2144-53.
**26-** Pechere JC, Hughes D, Kardas P, Cornaglia G. Non-compliance with antibiotic therapy for acute community infections: a global survey. International journal of Antimicrobial Agents, 2007;29:245-253
**27-**Pepys MB, Hirschfield GM. C-reactive protein: a critical update. Journal Clinical Investigation, 2003;111: 1805-1812.
**28-**Peterson JW. Bacterial pathogenesis. In: Medical Microbiology, 4th Edition, The University of Texas Medical Branch at Galveston: Galveston, TX, 1996.
**29-**Pham NA, Morrison A,JSchwock J, AvielRonen S, Iakovlev V, Tsao M.S., Ho J., Hedley D.W., Quantitative image analysis of immunohistochemical stains using a CMYK color model, Diagn Pathol Pathol,2007;2:8
**30-**Reinert RR, Low DE, Rossi F, Zhang X, Wattal C, Dowzicky MJ. Antimicrobial susceptibility among organisms from the Asia/Pacific Rim, Europe and Latin and North America collected as part of TEST and the in vitro activity of tigecycline. Journal of Antimicrobial Chemotherapy. 2007;60(5):1018-1029.
**31-**Richi H, Dhillon P, Clark J. ESBLs: A clear and present danger? Crit Care Res Pract. 2012; 2012: 625170.
**32-** Steart PS, Costerton JW. Antibiotic resistance of bacteria in biofilms, The Lancet,2001: 358:135-138
**33-** Theocharis AD, Skandalis SS; Gialeli C, Karamanos NK. Extracellular matrix structure Adv Drug Deliv Rev, 2015 [Epub ahead of print].
**34-** Thorne GM. Diagnosis of infectious diarrheal diseases. Infect Dis North Am. 1988;2(3):747-74
**35-** Tunkel AR, Hartman BJ, Kaplan SL, Kaufman BA, Roos KL; Scheld M, Whiteley RJ. Practise guidelines for the management of bacterial meningitis. Clinical Infectious Diseases, 2004; 39:1267-1284
**36-**Uma B, Prabhakar K, Rajendran S, Lakshmi Sarayu Y. Prevalence of extended spectrum beta lactamases in Salmonella species isolated from patients with acute gastroenteritis. Indian J Gastroenterol. 2010. 29:201-204
**37-** Walti LN, Conen A, Coward J, Jost GF, Trampuz A. Characteristics of infections associated with ventricular drains of cerebrospinal fluid. J Infect 2013;66(5):424-31.
**38-** Wolcott R, Costerton JW, Raoult D, Cutler SJ. The polymicrobial nature of biofilm infection. Clinical Microbiology and Infection, 2013; 19:107-112.
**39-**Xiao Z, Wilson C, Robertson HL, Roberts DJ, Ball CG, Jenne CN, Kirkpatrick Inflammatory mediators in intra-abdominal sepsis or injury - a scoping review. Critical Care, 2015; 19:1093-1094

## Claims

1. A device for quantitatively determining the presence or absence of one or more acute phase protein(s), such as Hepatocyte Growth Factor (HGF), in a biological sample from an individual, a presence of one or more acute phase protein(s) in said biological sample being indicative of an acute bacterial infection in said individual, said device comprising
a) at least one surface on which a composition comprising:
i) a proteoaminoglycan or dextran sulphate and
ii) toluidine blue (chemical formula: C₁₅H₁₆N₃S) has been immobilized,
b) optionally a solid support onto which said at least one surface may be attached,
wherein said at least one surface comprises a material suitable for immobilization of said composition comprising proteoaminoglycan or dextran sulphate and toluidine blue; and
wherein on at least one surface of a) the ratio of the amount of a proteoaminoglycan or dextran sulphate to the amount of toluidine blue in said composition on said at least one surface is selected from the group of ratios consisting of:
• about 100:1, such as about 90:1 to about 110:1 (surface 1);
• about 140:1, such as about 135:1 to about 145:1 (surface 2);
• about 165:1, such as about 160:1 to about 170:1 (surface 3);
• about 180:1, such as about 175:1 to about 185:1 (surface 4);
• about 200:1, such as about 190:1 to about 210:1 (surface 5); and
• about 225:1, such as about 220:1 to about 230:1 (surface 6).

2. The device of claim 1, said device comprising at least two surfaces on which a composition comprising a proteoaminoglycan or dextran sulphate and toluidine blue has been immobilized, wherein the ratio of the amount of a proteoaminoglycan or dextran sulphate to the amount of toluidine blue in the composition on said first surface is different than the ratio of the amount of proteoaminoglycan or dextran sulphate to the amount of toluidine blue in the composition on said second surface.

3. The device of claim 1 or 2, wherein said ratio for surface 1 is about 100:1, for surface 2 is about 138:1, for surface 3 is about 165:1, for surface 4 is about 180:1, for surface 5 is about 200:1 and/or said ratio for surface 6 is about 226:1.

4. The device of claim 2 or 3, said device comprising two or more, such as two, three, or four of the surfaces as defined in claims 1 or 3.

5. The device of any one of claims 1 to 4, wherein said device comprises surface 1 and surface 2.

6. The device of any one of claims 1 to 4, wherein said device comprises at least one, such as two or three of: surface 3, surface 4 and/or surface 5.

7. The device of any one of claims 1 to 4, wherein said device comprises surface 5 and surface 6.

8. The device of any one of the preceding claims, wherein said proteoaminoglycan is selected from the group consisting of Heparan Sulphate Proteoglycan and heparan sulphate.

9. The device of any one of claims 1 to 8, wherein said device is present in a diaper or a hand-held device for detection of an acute bacterial infection.

10. A kit of parts, said kit of parts comprising a device according to any one of claims 1 to 9, said kit further comprising a colour detecting means for quantitative determination of acute phase protein(s) detected by said device, and optionally instructions for use.

11. An *in vitro* method for quantitatively determining the presence or absence of one or more acute phase protein(s) in a biological sample from an individual, said presence of one or more acute phase protein(s) being indicative of an acute bacterial infection in said individual, said method comprising the steps of:
a) adding said biological sample to a device comprising at least one surface according to any one of claims 1 to 9,
b) incubating said biological sample on the surface of said device in step a), and thereafter,
c) comparing the colour of the surface with a reference obtained with known amounts of acute-phase proteins to determine the presence or absence of one or more acute phase protein(s) in said biological sample,
optionally wherein said acute phase protein is Hepatocyte Growth Factor, a derivative thereof or a protein related thereto, or calprotectin,
wherein optionally, determining the presence or absence of one or more acute phase protein(s) in said biological sample is used for monitoring a therapy, such as an antibiotic therapy

12. The *in vitro* method of claim 11, wherein said device comprises surface 1 and surface 2 as defined in claim 1 or 3.

13. The *in vitro* method of claim 11, wherein said device comprises at least one, such as one, two or three of: surface 3, surface 4 and surface 5 as defined in claim 1 or claim 3, and wherein said biological sample comprises sputum, broncho-alveolar lavage, a nasal secretion, cerebrospinal fluid, urine or renal secrete.

14. The *in vitro* method of claim 11, wherein said device comprises surface 5 and surface 6, as defined in claim 1 or claim 3 and wherein said biological sample comprises ulcer secretion.

15. A method for determining a suitable material for a surface as defined in claim 1, said method comprising the steps of:
a) soaking a material in a liquid solution comprising a composition comprising a proteoaminoglycan or dextran sulphate, and toluidine blue in any one of the ratios as defined in claim 1,
b) determining the colour of the material after soaking in the solution of step a),
wherein if the material substantially presents the colour of toluidine blue bound to proteoaminoglycan or dextran sulphate after soaking, said material is suitable for a surface as defined in claim 1, wherein the material will turn purple red or red when toluidine blue is bound to a proteoaminoglycan or dextran sulphate on said suitable material.

## Patentansprüche

1. Vorrichtung zur quantitativen Bestimmung der Anwesenheit oder Abwesenheit von einem oder mehreren Akute-Phase-Protein(en), wie dem Hepatozyten-Wachstumsfaktor (Hepatocyte Growth Factor; HGF), in einer biologischen Probe von einem Individuum, wobei die Anwesenheit eines Akute-Phase-Proteins oder mehrerer Akute-Phase-Proteine in der biologischen Probe auf eine akute bakterielle Infektion in dem Individuum hinweist, wobei die Vorrichtung umfasst:
a) mindestens eine Oberfläche, auf der eine Zusammensetzung, umfassend:
i) ein Proteoaminoglykan oder Dextransulfat und
ii) Toluidinblau (chemische Formel: C₁₅H₁₆N₃S) immobilisiert wurde,
b) gegebenenfalls einen festen Träger, auf dem die mindestens eine Oberfläche angebracht werden kann,
wobei die mindestens eine Oberfläche ein Material umfasst, das für die Immobilisierung der Zusammensetzung, die Proteoaminoglykan oder Dextransulfat und Toluidinblau umfasst, geeignet ist; und
wobei auf mindestens einer Oberfläche aus a) das Verhältnis der Menge eines Proteoaminoglykans oder von Dextransulfat zur Menge von Toluidinblau in der Zusammensetzung auf der mindestens einen Oberfläche ausgewählt ist aus der Gruppe von Verhältnissen bestehend aus:
• etwa 100:1, wie beispielsweise etwa 90:1 bis etwa 110:1 (Oberfläche 1);
• etwa 140:1, wie beispielsweise etwa 135:1 bis etwa 145:1 (Oberfläche 2);
• etwa 165:1, wie beispielsweise etwa 160:1 bis etwa 170:1 (Oberfläche 3);
• etwa 180:1, wie beispielsweise etwa 175:1 bis etwa 185:1 (Oberfläche 4);
• etwa 200:1, wie beispielsweise etwa 190:1 bis etwa 210:1 (Oberfläche 5); und
• etwa 225:1, wie beispielsweise etwa 220:1 bis etwa 230:1 (Oberfläche 6).

2. Vorrichtung nach Anspruch 1, wobei die Vorrichtung mindestens zwei Oberflächen umfasst, auf der eine Zusammensetzung, die ein Proteoaminoglykan oder Dextransulfat und Toluidinblau umfasst, immobilisiert wurde, wobei das Verhältnis der Menge eines Proteoaminoglykans oder von Dextransulfat zu der Menge von Toluidinblau in der Zusammensetzung auf der ersten Oberfläche ein unterschiedliches Verhältnis ist als das Verhältnis der Menge von Proteoaminoglykan oder Dextransulfat zu der Menge von Toluidinblau in der Zusammensetzung auf der zweiten Oberfläche.

3. Vorrichtung nach Anspruch 1 oder 2, wobei das Verhältnis für die Oberfläche 1 etwa 100:1 ist, für die Oberfläche 2 etwa 138:1 ist, für die Oberfläche 3 etwa 165:1 ist, für die Oberfläche 4 etwa 180:1 ist, für die Oberfläche 5 etwa 200:1 ist und/oder das Verhältnis für die Oberfläche 6 etwa 226:1 ist.

4. Vorrichtung nach Anspruch 2 oder 3, wobei die Vorrichtung zwei oder mehrere, wie beispielsweise zwei, drei oder vier der wie in Anspruch 1 oder 3 definierten Oberflächen umfasst.

5. Vorrichtung nach einem der Ansprüche 1 bis 4, wobei die Vorrichtung die Oberfläche 1 und die Oberfläche 2 umfasst.

6. Vorrichtung nach einem der Ansprüche 1 bis 4, wobei die Vorrichtung mindestens eine, wie beispielsweise zwei oder drei von: Oberfläche 3, Oberfläche 4 und/oder Oberfläche 5 umfasst.

7. Vorrichtung nach einem der Ansprüche 1 bis 4, wobei die Vorrichtung die Oberfläche 5 und die Oberfläche 6 umfasst.

8. Vorrichtung nach einem der vorangehenden Ansprüche, wobei das Proteoaminoglykan ausgewählt ist aus der Gruppe bestehend aus Heparansulfat-Proteoglykan und Heparansulfat.

9. Vorrichtung nach einem der Ansprüche 1 bis 8, wobei die Vorrichtung in einer Windel oder einer tragbaren Vorrichtung für den Nachweis einer akuten bakteriellen Infektion vorhanden ist.

10. Kit-of-Parts, wobei der Kit-of-Parts eine Vorrichtung nach einem der Ansprüche 1 bis 9 umfasst, wobei der Kit des Weiteren ein Farberkennungsmittel für die quantitative Bestimmung von (einem) durch die Vorrichtung nachgewiesenen Akute-Phase-Protein(en) umfasst, und gegebenenfalls Gebrauchshinweise.

11. *In vitro*-Verfahren zur quantitativen Bestimmung der Anwesenheit oder Abwesenheit von einem oder mehreren Akute-Phase-Protein(en) in einer biologischen Probe von einem Individuum, wobei die Anwesenheit eines Akute-Phase-Proteins oder mehrerer Akute-Phase-Proteine auf eine akute bakterielle Infektion in dem Individuum hinweist, wobei das Verfahren die Schritte umfasst:
a) Hinzufügen der biologischen Probe zu einer Vorrichtung, die mindestens eine Oberfläche umfasst, nach einem der Ansprüche 1 bis 9,
b) Inkubieren der biologischen Probe auf der Oberfläche der Vorrichtung in Schritt a), und danach
c) Vergleichen der Farbe der Oberfläche mit einer Referenz, die mit bekannten Mengen von Akute-Phase-Proteinen erhalten wurde, um die Anwesenheit oder Abwesenheit von einem oder mehreren Akute-Phase-Protein(en) in der biologischen Probe zu bestimmen,
wobei gegebenenfalls das Akute-Phase-Protein der Hepatozyten-Wachstumsfaktor, ein Derivat davon oder ein damit verwandtes Protein oder Calprotectin ist,
wobei gegebenenfalls das Bestimmen der Anwesenheit oder Abwesenheit von einem oder mehreren Akute-Phase-Protein(en) in der biologischen Probe für das Überwachen einer Therapie, wie beispielsweise einer Antibiotikatherapie, verwendet wird.

12. *In vitro*-Verfahren nach Anspruch 11, wobei die Vorrichtung die in Anspruch 1 oder 3 definierte Oberfläche 1 und Oberfläche 2 umfasst.

13. *In vitro*-Verfahren nach Anspruch 11, wobei die Vorrichtung mindestens eine, wie beispielsweise eine, zwei oder drei aus: Oberfläche 3, Oberfläche 4 und Oberfläche 5, wie in Anspruch 1 oder Anspruch 3 definiert, umfasst, und wobei die biologische Probe Sputum, bronchoalveoläre Lavage, ein Nasensekret, zerebrospinale Flüssigkeit, Urin oder Nierensekret umfasst.

14. *In vitro*-Verfahren nach Anspruch 11, wobei die Vorrichtung die wie in Anspruch 1 oder Anspruch 3 definierte Oberfläche 5 und Oberfläche 6 umfasst und wobei die biologische Probe ein Geschwürsekret umfasst.

15. Verfahren zur Bestimmung eines geeigneten Materials für eine wie in Anspruch 1 definierte Oberfläche, wobei das Verfahren die Schritte umfasst:
a) Einweichen eines Materials in einer flüssigen Lösung, die eine Zusammensetzung umfasst, die ein Proteoaminoglykan oder Dextransulfat und Toluidinblau in einem wie in Anspruch 1 definierten Verhältnis umfasst,
b) Bestimmen der Farbe des Materials nach dem Einweichen in der Lösung aus Schritt a), wobei im Falle, dass das Material nach dem Einweichen im Wesentlichen die Farbe von an Proteoaminoglykan oder Dextransulfat gebundenem Toluidinblau darstellt, das Material für eine wie in Anspruch 1 definierte Oberfläche geeignet ist, wobei sich das Material purpurrot oder rot färben wird, wenn Toluidinblau auf dem geeigneten Material an Proteoaminoglykan oder Dextransulfat gebunden ist.

## Revendications

1. Dispositif pour déterminer quantitativement la présence ou l'absence d'une ou de plusieurs protéines de phase aiguë, telles que le facteur de croissance des hépatocytes (HGF), dans un échantillon biologique provenant d'un individu, une présence d'une ou de plusieurs protéines de phase aiguë dans ledit échantillon biologique indiquant une infection bactérienne aiguë chez ledit individu, ledit dispositif comprenant
a) au moins une surface sur laquelle a été immobilisée une composition comprenant :
i) un protéoaminoglycane ou un sulfate de dextrane et
ii) du bleu de toluidine (formule chimique : C₁₅H₁₆N₃S),
b) éventuellement un support solide sur lequel ladite au moins une surface peut être fixée, dans lequel ladite au moins une surface comprend un matériau approprié pour l'immobilisation de ladite composition comprenant un protéoaminoglycane ou un sulfate de dextrane et du bleu de toluidine ; et
dans lequel sur au moins une surface de a), le rapport de la quantité d'un protéoaminoglycane ou d'un sulfate de dextrane sur la quantité de bleu de toluidine dans ladite composition sur ladite au moins une surface est choisi dans le groupe des rapports consistant en :
• environ 100:1, tel qu'environ 90:1 à environ 110:1 (surface 1) ;
• environ 140:1, tel qu'environ 135:1 à environ 145:1 (surface 2) ;
• environ 165:1, tel qu'environ 160:1 à environ 170:1 (surface 3) ;
• environ 180:1, tel qu'environ 175:1 à environ 185:1 (surface 4) ;
• environ 200:1, tel qu'environ 190:1 à environ 210:1 (surface 5) ; et
• environ 225:1, tel qu'environ 220:1 à environ 230:1 (surface 6).

2. Dispositif selon la revendication 1, ledit dispositif comprenant au moins deux surfaces sur lesquelles une composition comprenant un protéoaminoglycane ou un sulfate de dextrane et du bleu de toluidine a été immobilisée, dans lequel le rapport de la quantité d'un protéoaminoglycane ou d'un sulfate de dextrane sur la quantité de bleu de toluidine dans la composition sur ladite première surface est différent du rapport de la quantité d'un protéoaminoglycane ou d'un sulfate de dextrane sur la quantité de bleu de toluidine dans la composition sur ladite deuxième surface.

3. Dispositif selon la revendication 1 ou 2, dans lequel ledit rapport pour la surface 1 est environ de 100:1, pour la surface 2 est environ de 138:1, pour la surface 3 est environ de 165:1, pour la surface 4 est environ de 180:1, pour la surface 5 est environ de 200:1 et/ou ledit rapport pour la surface 6 est environ de 226:1.

4. Dispositif selon la revendication 2 ou 3, ledit dispositif comprenant deux ou plus, comme deux, trois ou quatre des surfaces telles que définies dans les revendications 1 ou 3.

5. Dispositif selon l'une quelconque des revendications 1 à 4, dans lequel ledit dispositif comprend la surface 1 et la surface 2.

6. Dispositif selon l'une quelconque des revendications 1 à 4, dans lequel ledit dispositif comprend au moins une, comme deux ou trois parmi : la surface 3, la surface 4 et/ou la surface 5.

7. Dispositif selon l'une quelconque des revendications 1 à 4, dans lequel ledit dispositif comprend la surface 5 et la surface 6.

8. Dispositif selon l'une quelconque des revendications précédentes, dans lequel ledit protéoaminoglycane est choisi dans le groupe consistant en un protéoglycane sulfate d'héparane et un sulfate d'héparane.

9. Dispositif selon l'une quelconque des revendications 1 à 8, dans lequel ledit dispositif est présent dans une couche-culotte ou un dispositif portatif pour la détection d'une infection bactérienne aiguë.

10. Kit d'éléments, ledit kit d'éléments comprenant un dispositif selon l'une quelconque des revendications 1 à 9, ledit kit comprenant en outre un moyen de détection de couleur pour la détermination quantitative d'une ou de plusieurs protéines de phase aiguë détectées par ledit dispositif, et éventuellement des instructions d'utilisation.

11. Procédé *in vitro* pour déterminer quantitativement la présence ou l'absence d'une ou de plusieurs protéines de phase aiguë dans un échantillon biologique provenant d'un individu, ladite présence d'une ou de plusieurs protéines de phase aiguë indiquant une infection bactérienne aiguë chez ledit individu, ledit procédé comprenant les étapes suivantes :
a) l'ajout dudit échantillon biologique à un dispositif comprenant au moins une surface selon l'une quelconque des revendications 1 à 9,
b) la mise en incubation dudit échantillon biologique sur la surface dudit dispositif dans l'étape a), puis
c) la comparaison de la couleur de la surface à une référence obtenue avec des quantités connues de protéines de phase aiguë pour déterminer la présence ou l'absence d'une ou de plusieurs protéines de phase aiguë dans ledit échantillon biologique,
éventuellement dans lequel ladite protéine de phase aiguë est le facteur de croissance des hépatocytes, un dérivé de celui-ci ou une protéine apparentée à celui-ci, ou la calprotectine,
dans lequel éventuellement la détermination de la présence ou de l'absence d'une ou de plusieurs protéines de phase aiguë dans ledit échantillon biologique est utilisée pour surveiller une thérapie, telle qu'une thérapie antibiotique.

12. Procédé *in vitro* selon la revendication 11, dans lequel ledit dispositif comprend la surface 1 et la surface 2 telles que définies dans la revendication 1 ou 3.

13. Procédé *in vitro* selon la revendication 11, dans lequel ledit dispositif comprend au moins une, comme une, deux ou trois parmi : la surface 3, la surface 4 et la surface 5 telles que définies dans la revendication 1 ou la revendication 3, et dans lequel ledit échantillon biologique comprend un crachat, un lavage broncho-alvéolaire, une sécrétion nasale, un liquide céphalo-rachidien, de l'urine ou une sécrétion rénale.

14. Procédé *in vitro* selon la revendication 11, dans lequel ledit dispositif comprend la surface 5 et la surface 6, telles que définies dans la revendication 1 ou la revendication 3 et dans lequel ledit échantillon biologique comprend une sécrétion d'ulcère.

15. Procédé pour déterminer une matière appropriée pour une surface telle que définie dans la revendication 1, ledit procédé comprenant les étapes suivantes :
a) le trempage d'une matière dans une solution liquide comprenant une composition comprenant un protéoaminoglycane ou un sulfate de dextrane, et du bleu de toluidine dans l'un quelconque des rapports tels que définis dans la revendication 1,
b) la détermination de la couleur de la matière après trempage dans la solution de l'étape a), dans lequel si la matière présente sensiblement la couleur du bleu de toluidine lié à un protéoaminoglycane ou à un sulfate de dextrane après trempage, ladite matière est appropriée pour une surface telle que définie dans la revendication 1, dans lequel la matière deviendra rouge violet ou rouge quand le bleu de toluidine se lie à un protéoaminoglycane ou à un sulfate de dextrane sur ladite matière appropriée.
